# FASCICULE DE BREVET EUROPEEN

(11) **EP 1 239 938 B1**
(45) Date de publication et mention de la délivrance du brevet: **11.03.2009**
(21) Numéro de dépôt: 00988935.3
(22) Date de dépôt: 15.12.2000
(51) Int. Cl.: B01D 46/30, B01J 2/00, A61K 9/16

(54) **PROCEDE ET DISPOSITIF DE CAPTAGE DE FINES PARTICULES PAR PERCOLATION DANS UN LIT DE GRANULES**
VERFAHREN UND VORRICHTUNG ZUR ENTFERNUNG VON FEINTEILIGEN PARTIKELN DURCH PERKOLATION IM SCHÜTTGUT
METHOD FOR CAPTURING FINE PARTICLES BY PERCOLATION IN A BED OF GRANULES

(30) Priorité: 15.12.1999 FR 9915834
(43) Date de publication de la demande: 18.09.2002
(73) Titulaire: Separex Société Anonyme, 54250 Champigneulles (FR)
(72) Inventeur: PERRUT, Michel, F-54000 Nancy (FR)
(74) Mandataire: Puiroux, Guy
(86) Numéro de dépôt international: PCT/FR2000/003558
(87) Numéro de publication internationale: WO 2001/043853

(56) Documents cités:
- EP-A- 0 865 819
- WO-A-97/31691
- DE-A- 3 719 549
- GB-A- 2 252 059
- US-A- 4 822 497

## Description

La présente invention concerne un procédé permettant d'assurer le captage de particules solides de grande finesse générées par un procédé mettant en oeuvre un fluide à pression supercritique ainsi qu'un dispositif permettant de mettre en oeuvre ce procédé.

De nombreuses industries utilisent des solides sous forme pulvérulente. C'est en particulier le cas des industries fabriquant des peintures, des produits cosmétiques et dermatologiques, et des produits pharmaceutiques. Ainsi, l'industrie pharmaceutique, mais également l'industrie des cosmétiques, requiert de nouvelles formes galéniques afin d'améliorer le service rendu par les molécules d'intérêt thérapeutique ou dermatologique. En particulier, elle recherche les moyens de réaliser une dissolution rapide de ces molécules, qui se présentent sous forme de poudre solide dans les conditions habituelles, au sein des fluides biologiques tels que le sang ou la lymphe. Pour ce faire, il est nécessaire soit de modifier la morphologie du solide, soit de diminuer très fortement la granulométrie de la poudre, soit de combiner ces deux actions. De nombreux travaux sont également conduits en vue d'élaborer des médicaments complexes permettant une absorption lente et régulière de la molécule active (médicament-retard).

On sait, par de nombreux brevets et publications scientifiques, que l'on peut obtenir des microparticules, d'une granulométrie généralement comprise entre 1µm et 10µm, et des nanoparticules d'une granulométrie généralement comprise entre 0,1µm et 1µm, en utilisant des procédés mettant en oeuvre des fluides supercritiques.

Les fluides supercritiques, et particulièrement le dioxyde de carbone supercritique, sont largement utilisés pour réaliser des poudres très fines susceptibles de se dissoudre très rapidement par ingestion par les voies respiratoires. Les fluides supercritiques sont également utilisés pour obtenir des particules complexes constituées de mélanges de différentes morphologies du principe actif et d'un excipient, tel que des microsphères ou des microcapsules.

On rappellera tout d'abord ce qu'est un tel fluide supercritique.

On sait en effet que les corps sont généralement connus sous trois états, à savoir solide, liquide ou gazeux et que l'on passe de l'un à l'autre en faisant varier la température et/ou la pression. Or il existe un point au-delà duquel on peut passer de l'état liquide à l'état gaz ou vapeur sans passer par une ébullition ou à l'inverse par une condensation, mais de façon continue : ce point est appelé le point critique.

On sait également qu'un fluide en état supercritique, c'est-à-dire un fluide qui est dans un état caractérisé soit par une pression et une température respectivement supérieures à la pression et à la température critiques dans le cas d'un corps pur, soit par un point représentatif (pression, température) situé au-delà de l'enveloppe des points critiques représentés sur un diagramme (pression, température) dans le cas d'un mélange, présente, pour de très nombreuses substances, un pouvoir solvant élevé sans commune mesure avec celui observé dans ce même fluide à l'état de gaz comprimé. Il en est de même des liquides dits « subcritiques » c'est-à-dire des liquides qui se trouvent dans un état caractérisé soit par une pression supérieure à la pression critique et par une température inférieure à la température critique dans le cas d'un corps pur, soit par une pression supérieure aux pressions critiques et une température inférieure aux températures critiques des composants dans le cas d'un mélange (cf l'article de Michel PERRUT - Les Techniques de l'Ingénieur « Extraction par fluide supercritique, J 2 770 - 1 à 12, 1999 »).

Les variations importantes et modulables du pouvoir solvant des fluides supercritiques sont d'ailleurs utilisées dans de nombreux procédés d'extraction (solide/fluide), de fractionnement (liquide/fluide), de chromatographie analytique ou préparative, de traitement des matériaux (céramiques, polymères) et de génération de particules. Des réactions chimiques ou biochimiques sont également réalisées dans de tels solvants. Il est à noter que les propriétés physico-chimiques du dioxyde de carbone ainsi que ses paramètres critiques (pression critique : 7,4 MPa et température critique : 31°C) en font le solvant préféré dans de nombreuses applications, d'autant qu'il ne présente pas de toxicité et est disponible à très bas prix en très grande quantité. Solvant non polaire, le dioxyde de carbone porté à pression supercritique est parfois additionné d'un co-solvant constitué notamment par un solvant organique polaire qui a pour fonction de modifier le pouvoir solvant de façon notable, surtout vis-à-vis de molécules présentant une certaine polarité, l'éthanol étant souvent utilisé à cette fin. Toutefois, certains composés sont plus favorablement extraits par un hydrocarbure léger ayant de 2 à 5 atomes de carbone, et plus favorablement, de 2 à 4 atomes de carbone, à pression supercritique.

Parmi les procédés permettant d'obtenir de très fines particules au moyen d'un fluide à pression supercritique, on retiendra particulièrement le procédé connu sous la désignation de « RESS » et suivant lequel on détend très rapidement une solution du produit à atomiser dans un fluide supercritique, et le procédé anti-solvant du type des procédés dits « SAS », « SEDS », « PCA », « ASES », consistant à pulvériser une solution du produit dans un solvant organique ou aqueux au sein d'un courant de fluide en état supercritique.

Ces procédés permettent l'obtention d'une poudre formée de particules très fines, qui sont dispersées au sein d'un courant gazeux à faible pression (procédé RESS), technique décrite dans le document EP-A-0865819, ou à pression élevée (procédé SAS) technique décrite dans le document WO-A-9731691.

La collecte de ces particules est ensuite une opération très délicate, surtout lorsque l'on souhaite mettre en oeuvre des productions importantes. En effet, à l'échelle du laboratoire, les particules générées sont captées par filtration sur un organe filtrant tissé ou non-tissé généralement disposé au fond du récipient où est réalisée la génération des particules. La récupération du filtre chargé de particules et la collecte des particules nécessitent donc la dépressurisation complète de ce récipient, son ouverture et la manipulation du filtre. Cette procédure n'est pas compatible avec les exigences d'hygiène et de sécurité en vigueur dans l'industrie pharmaceutique, car une partie des fines particules se retrouvent dans l'atmosphère avec les risques d'inhalation par le personnel présent, et une contamination du médicament ainsi atomisé est également à craindre. Enfin, il est évident qu'une telle procédure est coûteuse et peu adaptée à une extrapolation à grande échelle.

On connaît bien entendu diverses méthodes permettant de collecter de fines particules au sein d'un courant gazeux à une pression voisine de la pression atmosphérique notamment dans le domaine du dépoussiérage. Les différents procédés et équipements de dépoussiérage utilisés actuellement sont adaptés à la taille des particules à capter. On retiendra :
- Les dispositifs inertiels, tels que les chicanes et les cyclones, qui sont efficaces pour capter des particules dont le diamètre est supérieur à 10 ou 20µm ;
- Les dispositifs électrostatiques, tels que les dépoussiéreurs utilisés pour le traitement des fumées de chaudières à charbon, qui sont des appareils complexes, efficaces pour capter des particules très fines d'un diamètre supérieur à 1µm environ ;
- Les laveurs de gaz de différentes conceptions qui sont adaptés au captage de particules selon leur diamètre, les plus efficaces étant les laveurs à tuyère Venturi qui permettent de capter des particules de diamètres submicroniques ;
- Les filtres constitués de matériaux filtrants tissés ou non tissés qui permettent de capter les particules les plus fines y compris celles dont le diamètre est compris entre 0,1 et 1 µm.

Chacune de ces techniques a toutefois des limitations selon les caractéristiques des particules à capter.

Dans le cas de fines particules à usage pharmaceutique ou cosmétique, il est clair que les dispositifs inertiels ne sont pas assez efficaces et que les dispositifs électrostatiques ne sont pas utilisables pour des raisons de coût et de sécurité. Il ne reste donc que les laveurs et les filtres.

Les laveurs ne peuvent être mis en oeuvre que si l'on accepte de collecter les particules sous forme d'une dispersion au sein d'un liquide où elles sont rigoureusement insolubles ; il est rare que l'utilisation ultérieure permette la mise en oeuvre d'une telle dispersion.

Les filtres présentent aussi un inconvénient notoire, dans la mesure où la récupération des particules qu'ils ont fixées ainsi que leur réutilisation ultérieure éventuelle, sont des opérations particulièrement difficiles à effectuer dès lors que l'on souhaite respecter les règles imposées dans l'industrie pharmaceutique.

La présente invention a pour but de proposer un procédé, ainsi que des moyens de mise en oeuvre de ce procédé, qui permette de capter facilement de telles particules et qui de plus se prête à un fonctionnement en continu à l'échelle industrielle.

La présente invention a ainsi pour objet un procédé de captage de très fines particules générées par un procédé mettant en oeuvre un fluide à pression supercritique caractérisé en ce que l'on fait percoler, dans une enceinte de traitement, ledit fluide chargé des particules à travers un lit récepteur constitué de granules. Le fluide à pression supercritique sera de préférence constitué de dioxyde de carbone.

Ces très fines particules seront ainsi piégées principalement dans la porosité extra-granulaire mais pourront également diffuser à l'intérieur même des granules lorsqu'ils présentent une porosité importante constituée de pores de diamètres supérieurs à ceux des particules générées.

Dans un mode de mise en oeuvre particulièrement intéressant de l'invention, les particules seront constituées d'un principe actif et les granules seront constituées d'un excipient destiné à fixer celui-ci. On pourra récupérer le lit granulaire chargé de particules (principe actif) et l'utiliser directement pour confectionner des comprimés, emplir des gélules ou toute autre présentation destinée à un usage thérapeutique en pharmacie humaine ou vétérinaire, cosmétique ou phytosanitaire.

La présente invention permet de réaliser un captage en continu des particules et pour ce faire on alimentera l'enceinte de traitement par des granules contenus dans un récipient de stockage formant sas par rapport à ladite enceinte. De même les granules traités contenus dans l'enceinte de traitement seront recueillis dans un réservoir formant sas par rapport à l'enceinte.

Par ailleurs le débit d'alimentation de l'enceinte de traitement par les granules contenus dans le récipient de stockage sera voisin du débit de prélèvement réalisé dans ladite enceinte.

La présente invention a également pour objet un dispositif de captage comprenant une enceinte de captage pour fines particules générées grâce à un procédé mettant en oeuvre un fluide à pression supercritique chargé des particules caractérisé en ce qu'elle comporte, à sa partie supérieure, des moyens d'alimentation en particules à capter et, à sa partie inférieure, un lit de granules destinés à fixer les particules par percolation du fluide à travers le lit de granules, et des moyens d'alimentation et d'évacuation d'un fluide à pression supercritique destinés à véhiculer lesdites particules.

Préférentiellement, la partie inférieure de l'enceinte de captage formera une trémie convergente vers le bas qui sera en communication avec des moyens de réception/stockage pouvant former un sas par rapport à l'enceinte de traitement.

On décrira ci-après, à titre d'exemple non limitatif, des formes d'exécution de la présente invention, en référence au dessin annexé sur lequel :
La figure 1 est un schéma de principe d'une installation de production de particules et de captage suivant l'invention.
La figure 2 est un schéma montrant le détail des moyens de captage des particules mis en oeuvre dans l'installation représentée sur la figure 1.
La figure 3 est une variante du mode de mise en oeuvre de l'invention représenté sur la figue 2.

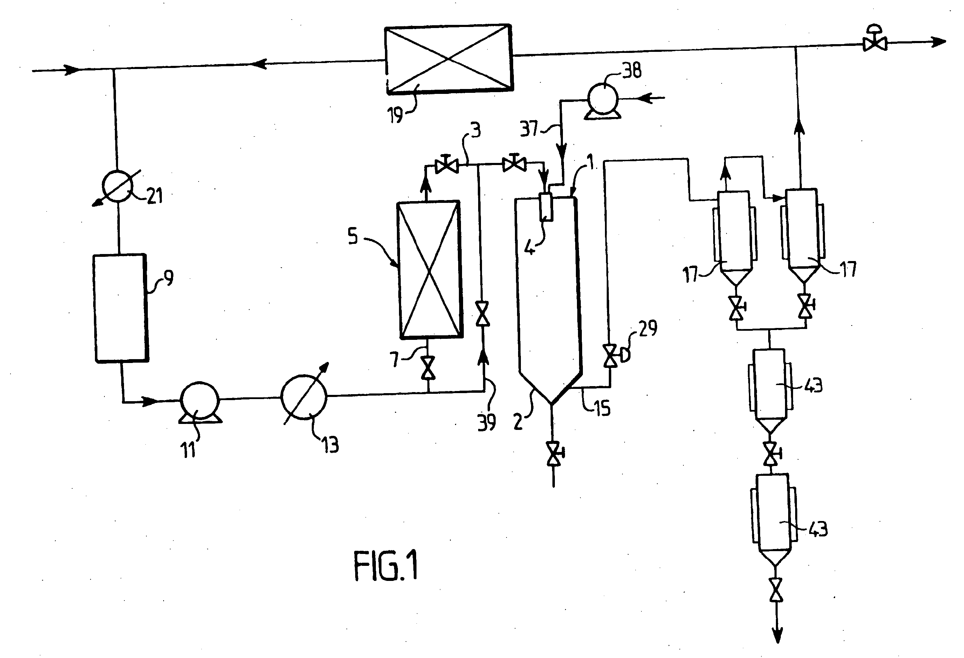

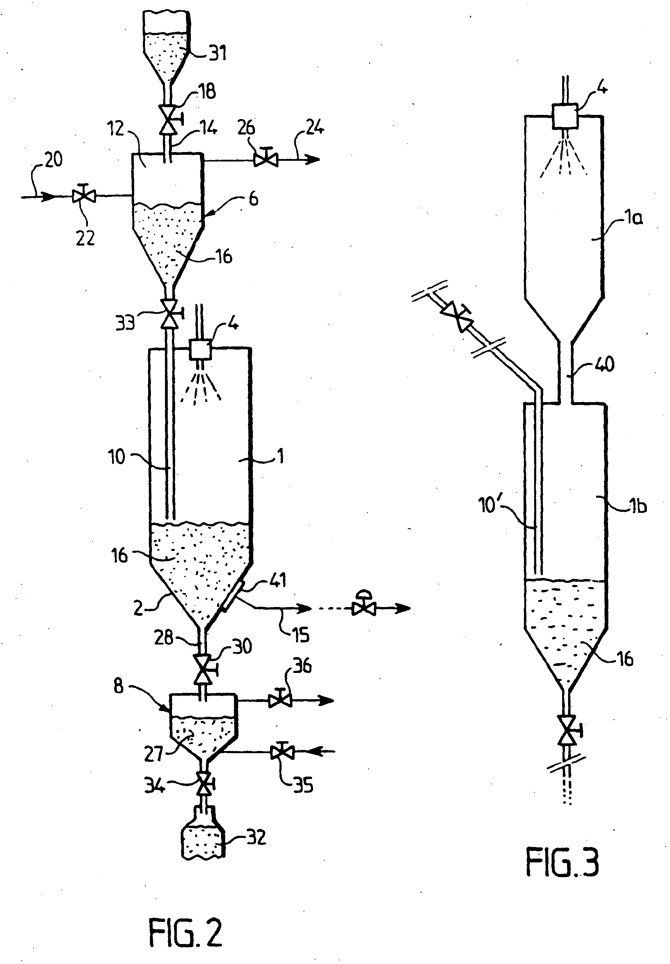

On a représenté sur la figure 1 un dispositif de production et de captage de particules extra fines suivant l'invention. Cette installation est essentiellement constituée d'une chambre d'atomisation 1, représentée de façon détaillée sur la figure 2, qui est reliée par une canalisation 3 à la partie supérieure, ou sortie, d'un extracteur 5 ou, par une canalisation 37, à une pompe d'injection de liquide 38.

Lorsque le procédé de génération de particules est de type RESS, l'extracteur 5 est alimenté à sa base par une canalisation 7 reliée à un réservoir de stockage 9 de gaz liquéfié par l'intermédiaire d'une pompe à membrane 11 et d'un échangeur 13 qui permettent de porter le gaz liquéfié à la pression et à la température souhaitées.

Lorsque le procédé de génération de particules est de type anti-solvant, l'extracteur 5 n'est pas utilisé et le fluide issu de l'échangeur 13 est directement alimenté à la chambre d'atomisation 1 via la canalisation 39, la solution du produit à atomiser dans un solvant organique ou aqueux étant introduit dans la partie supérieure de la chambre d'atomisation 1 via la canalisation 37 et la pompe 38.

Plus précisément la chambre d'atomisation 1 est constituée, ainsi que représenté sur la figure 2, par un récipient tubulaire d'axe vertical qui se termine à sa base par un fond conique 2 d'angle au sommet de l'ordre de 45°. Cette chambre d'atomisation 1 comporte, à sa partie supérieure, une buse d'injection 4 alimentée par la canalisation 3 reliée à l'extracteur 5, et à sa partie inférieure une sortie du fluide à pression supercritique formée d'un pastille filtre 41 en métal fritté en communication avec la canalisation 15.

La chambre d'atomisation 1 contient des granules 16 destinés à fixer les particules de très faible dimension. L'alimentation en granules 16 de la chambre d'atomisation 1, ainsi que la récupération des granules traités dans celle-ci sont réalisées par l'intermédiaire de deux systèmes de sas respectifs, à savoir un sas d'alimentation 6 et un sas de récupération 8.

Le sas d'alimentation 6 est constitué d'un tube 10 qui pénètre dans la chambre 1 par la partie supérieure de celle-ci et qui est relié à la partie inférieure d'une enceinte étanche 12 avec interposition d'une vanne 33. L'enceinte 12 est alimentée à sa partie supérieure par une canalisation 14 reliée à une trémie 31 d'alimentation en granules par l'intermédiaire d'une vanne à solide 18. La chambre 12 est par ailleurs réunie d'une part à une alimentation en fluide sous pression par une canalisation 20 avec interposition d'une vanne 22 et d'autre part à l'extérieur par une canalisation 24 avec interposition d'une vanne 26.

Dans un tel mode de mise en oeuvre de l'invention, le lit granulaire sera constitué de granules 16 d'une forme et d'une taille telles que l'on pourra récupérer facilement le lit chargé de particules, notamment chargées de principe actif, par un système de vannes habituellement utilisé pour récupérer des solides.

De même, le sas de récupération 8 est constitué d'une enceinte inférieure étanche 27 qui est reliée, par sa partie supérieure, à la base de la partie conique 2 de la chambre d'atomisation 1 par un tube 28 avec interposition d'une vanne de solide 30. La partie inférieure de l'enceinte 27 est réunie quant à elle à un récipient de récupération 32 avec interposition d'une vanne de solide 34. Cette enceinte 27 peut également être mise en communication via une vanne 35 avec le circuit de fluide à pression supercritique en aval de l'échangeur 13 et via une vanne 29 avec les séparateurs 17, de façon à permettre un strippage de la poudre collectée par un courant de fluide à pression supercritique.

On décrira tout d'abord le fonctionnement global de l'installation puis le fonctionnement spécifique de la chambre d'atomisation 1 et de ses moyens d'alimentation et de récupération des granules 16.

Suivant une technique connue, par exemple ici la technique dite RESS, le produit que l'on souhaite atomiser est disposé dans l'extracteur 5 et l'on fait percoler dans celui-ci un fluide à pression supercritique, constitué notamment par du dioxyde de carbone, qui est stocké dans le réservoir 9. Le fluide est porté à la pression de travail par la pompe à membrane 11 et à la température de travail par l'échangeur de chaleur 13. Le fluide à pression supercritique ayant dissous une certaine concentration du produit, il est admis dans la chambre d'atomisation 1 au travers de la buse de pulvérisation 4, à travers laquelle il est brutalement détendu, engendrant la formation de particules qui viennent se fixer sur les granules disposés dans la chambre 1. Le fluide est ensuite évacué à l'atmosphère ou, éventuellement recomprimé et recyclé.

Si au lieu de la technique RESS, on génère les particules par une autre technique connue, par exemple le procédé d'atomisation anti-solvant, on alimente directement la chambre d'atomisation 1 par le fluide à pression supercritique provenant du réservoir 9 via la pompe 11 et l'échangeur 13 et l'on pulvérise une solution du produit que l'on souhaite atomiser, que l'on aura préalablement dissous dans un solvant organique ou aqueux, dans la chambre d'atomisation 1 via la buse 4, la canalisation 37 et la pompe 38. Les particules ainsi générées viennent se fixer sur les granules disposés dans la chambre 1. Le fluide à pression supercritique est ensuite partiellement détendu à la pression de recyclage et réchauffé afin d'assurer la vaporisation du fluide et la séparation de la majeure partie du solvant utilisée pour élaborer la solution initiale du produit à atomiser. Ce solvant est récupéré dans les séparateurs cycloniques 17, puis soutiré à pression atmosphérique au travers de sas 43. Le fluide débarrassé de la majeure partie de solvant est recyclé, après purification éventuelle sur le lit d'absorbant 19, généralement constitué de charbon actif, puis liquéfié dans le condenseur 21, et récupéré dans le réservoir de fluide 9.

Les moyens d'alimentation et de récupération des granules dont la chambre d'atomisation 1 est équipée sont particulièrement intéressants dans la mesure où ils permettent un fonctionnement en continu de l'ensemble de l'installation, alors que dans les dispositifs de l'état antérieur de la technique, où les particules étaient récupérées sur un filtre disposé à l'intérieur de la chambre d'atomisation, il était nécessaire, préalablement à cette récupération, de réaliser une décompression complète de la chambre d'atomisation et l'ouverture de cette dernière.

Pour admettre les granules 16 à l'intérieur de la chambre d'atomisation 1, on recueille tout d'abord ceux-ci dans la l'enceinte supérieure 12 par ouverture de la vanne 18, les autres vannes en relation avec cette enceinte 12 étant alors fermées. On ferme ensuite la vanne 18 et on admet le fluide sous pression par la canalisation 20, en ouvrant la vanne 22, les autres vannes en relation avec cette enceinte étant fermées. Lorsque la pression dans cette enceinte atteint une valeur légèrement supérieure à celle régnant dans la chambre d'atomisation , on ferme la vanne 22 et on ouvre la vanne 33, si bien que les granules sont propulsés sous pression dans la chambre 1. Ensuite on ferme la vanne 33 et on ouvre la vanne 24 afin de dépressuriser l'enceinte 12 en vue d'un nouveau cycle de remplissage.

Une fois l'opération de captage des fines particules terminée, lorsque l'on souhaite récupérer les granules traités dans la chambre 1, on ouvre la vanne 30 et l'on récupère dans l'enceinte inférieure 27 les granules traités. Après fermeture de la vanne 30 et ouverture de la vanne 34 on récupère dans le récipient 32 les granules traités.

Lorsque le procédé de génération de particules est du type anti-solvant, il est préférable d'opérer le strippage du solvant organique, ayant servi à préparer la solution initiale du produit à atomiser, présent sur les granules et particules ainsi collectés, cette opération pouvant être avantageusement réalisée par balayage des granules par un courant de fluide à pression supercritique entrant par la vanne 35 et sortant par la vanne 36.

Suivant l'invention on réglera, par pesée du mélange recueilli, le débit de soutirage des granules de façon qu'il soit égal au débit d'entrée et que le volume du lit granulaire dans la chambre 1 soit ainsi maintenu à une valeur constante.

Il est ainsi possible de générer les particules de façon continue, de les capter et de les récupérer sans qu'il soit nécessaire, entre le traitement de deux lots successifs, d'être contraint de mettre la chambre de traitement à la pression atmosphérique. Par ailleurs on a constaté que le fait de travailler en continu permettait d'obtenir des lots de produit très homogènes à la différence de ceux obtenus suivant l'état antérieur de la technique c'est-à-dire en fonctionnement par lot classique.

### Exemple 1

On a utilisé l'installation, précédemment décrite dotée d'une chambre d'atomisation de volume total de 8 litres, pour extraire de la caféine par un fluide à pression supercritique et générer des particules fines par détente de ce fluide selon la technique RESS, ce fluide étant constitué de dioxyde de carbone à pression supercritique à pression de 30 Mpa et à une température de 60°C et un débit de 14 kg/h. Les particules ont été captées sur un lit de granules d'excipient, constitués de poudre d'hydroxypropylcellulose et de silice, 90% des granules ayant un diamètre compris entre 100µm et 300µm et se présentant comme une poudre coulant facilement. Ce lit, constitué de 800 g de granules a été mis en place au démarrage de l'installation, et il a été alimenté de 90 g de granules toutes les douze minutes tout en soutirant 100 g de solides toutes les douze minutes également, de façon que la masse de granules présente dans la chambre soit à peu près constante. Les cinq premiers lots de poudre ont été éliminés puis, sur chacun des lots suivants récupérés, on a effectué une analyse de la teneur en caféine par chromatographie en phase liquide HPLC après dissolution de la caféine dans l'eau.

On a constaté une excellente reproductibilité de la teneur en caféine de chaque lot obtenu lors d'une opération ayant duré 8 h 30, cette teneur restant comprise entre 10,5 et 11,8 g de caféine pour 100g de mélange.

### Exemple 2

Dans un second exemple de mise en oeuvre de l'invention et en utilisant la même installation, on a généré des particules très fines de tétracycline selon le procédé anti-solvant SAS, dans lequel on a pulvérise une solution de 5% en masse de tétracycline dans la N-méthylpyrrolidone avec un débit de 0,6 kg/h dans un courant de 15 kg/h de dioxyde dé carbone à pression supercritique, à savoir 18 MPa et 45°C.

On a capté les particules dans un lit de granules d'excipient constitués d'un mélange de poudres d'hydroxypropylcellulose (à 50% en poids) et de silice (à 50% en poids). 90 % des granules possédaient un diamètre compris entre 100µm et 300µm, si bien qu'ils se présentaient comme une poudre coulant facilement. Ce lit, constitué de 800g de granules, a été mis en place au démarrage de l'installation, et il a été alimenté de 100g de granules toutes les quinze minutes par le sas 6 tout en soutirant 100g de solide toutes les quinze minutes également par le sas 8, de façon que la masse de granules présente dans la chambre soit à peu près constante.

Suivant l'invention, à chaque cycle de prélèvement, la poudre a été soutirée, par le sas 8 et strippée pendant 10 minutes par un courant de dioxyde de carbone à 18 Mpa, à une température de 45°C et avec un débit de 3kg/h. Le sas a ensuite été décomprimé jusqu'à la pression atmosphérique avant le soutirage final de la poudre obtenue.

Les six premiers lots de poudre ainsi récupérés sont jetés. Chaque lot suivant a été récupéré séparément et la teneur en tétracycline a été déterminée par chromatographie en phase liquide après dissolution de la tétracycline dans l'eau.

On a constaté une excellente reproductibilité de la teneur de chaque lot lors d'une opération ayant duré 8 heures, cette teneur restant comprise entre 7,2 et 7,9% dans la poudre finale. La teneur de cette poudre en N-méthylpyrrolidone, déterminée par chromatographie en phase gazeuse de la phase aqueuse obtenue par agitation prolongée sous ultrasons de la poudre est restée inférieure à 100 ppm pour tous les lots.

Dans un mode de mise en oeuvre de l'invention représenté sur la figure 3, on a scindé la chambre d'atomisation 1 en deux chambres distinctes, savoir une chambre d'atomisation proprement dite où sont produites les fines particules et une chambre de captage où les fines particules produites sont captées par les granules.

Plus précisément l'installation comprend ainsi une chambre d'atomisation la qui est de même forme que celle représentée sur la figure 2, et qui est en communication par son fond et une canalisation 40 avec la partie supérieure d'une chambre de captage 1b de même forme, qui contient le lit de granules 16. L'alimentation en granules de là chambre de captage 1b est assurée par une conduite 10' reliée à un sas d'alimentation du type de celui représenté sur la figure 2.

Un tel mode de mise en oeuvre est particulièrement intéressant dans le domaine de l'exploitation industrielle dans la mesure où il permet de mettre en oeuvre plusieurs chambres d'atomisation et plusieurs chambres de captage, utilisées et nettoyées successivement.

Les expériences conduites dans des conditions initiales identiques à celles décrites dans les exemples précédents ont montré que les résultats obtenus étaient tout à fait comparables à la différence près que la mise en régime s'est révélée plus longue en raison du dépôt initial de particules fines sur le cône inférieur de la chambre d'atomisation.

## Revendications

1. Procédé de captage de très fines particules, comprenant une étape de génération des particules au moyen d'un fluide à pression supercritique, **caractérisé en ce que** le procédé comprend en outre, dans une enceinte de captage (1, 1b), une étape de percolation, à travers un lit récepteur constitué de granules, du fluide chargé des particules générées lors de l'étape précédente.

2. Procédé suivant la revendication 1 **caractérisé en ce que** les particules sont constituées d'un principe actif et les granules sont constitués d'un excipient destiné à fixer celui-ci.

3. Procédé suivant l'une des revendications 1 ou 2 **caractérisé en ce qu'**on utilise un fluide à pression supercritique constitué de dioxyde de carbone.

4. Procédé suivant l'une des revendications précédentes **caractérisé en ce qu'**on alimente l'enceinte de captage (1,1b) par des granules (16) contenus dans un récipient de stockage (12) formant sas par rapport à ladite enceinte (1,1b).

5. Procédé suivant la revendication 4 **caractérisé en ce qu'**on prélève les granules (16) traités contenus dans l'enceinte de captage (1, 1b) pour les recueillir dans un récipient (27) formant sas par rapport à l'enceinte (1, 1b).

6. Procédé suivant l'une des revendications 4 ou 5 **caractérisé en ce que** le débit d'alimentation de l'enceinte de captage (1, 1b) par les granules (16) contenus dans le récipient de stockage (12) est voisin du débit de prélèvement réalisé dans ladite enceinte (1, 1b).

7. Dispositif de captage pour fines particules générées grâce à un procédé mettant en oeuvre un fluide à pression supercritique chargé des particules, **caractérisé en ce qu'**il comporte une enceinte de captage (1, 1b) pourvue à sa partie supérieure, de moyens d'alimentation en particules à capter et, à sa partie inférieure, un lit de granules destinés à fixer les particules par percolation du fluide à travers le lit de granules, et des moyens d'alimentation et d'évacuation d'un fluide à pression supercritique destinés à véhiculer lesdites particules.

8. Dispositif de captage suivant la revendication 7 **caractérisé en ce que** la partie inférieure de l'enceinte de captage (1, 1b) forme une trémie convergente vers le bas qui est en communication avec des moyens de réception/stockage (8).

9. Dispositif de captage suivant la revendication 8 **caractérisé en ce que** les moyens de réception/stockage (8) forment un sas par rapport à l'enceinte de captage (1).

10. Dispositif de captage suivant l'une des revendications 8 ou 9 **caractérisé en ce que** les moyens d'alimentation (6) en particules à capter forment un sas par rapport à l'enceinte de captage (1).

## Claims

1. A method for capturing very fine particles, comprising a step for generating particles by means of a fluid at a supercritical pressure, **characterized in that** the method further comprises in a capture enclosure (1, 1b), a step for percolating the fluid loaded with the particles generated during the preceding step, through a receiving bed consisting of granules.

2. The method according to claim 1, **characterized in that** the particles consist of an active ingredient and the granules consist of an excipient intended to fix the latter.

3. The method according to any of claims 1 or 2, **characterized in that** a fluid at a supercritical pressure consisting of carbon dioxide is used.

4. The method according to any of the preceding claims, **characterized in that** the capture enclosure (1, 1b) is fed with granules (16) contained in a storage container (12) forming an airlock relatively to said enclosure (1, 1b).

5. The method according to claim 4, **characterized in that** the treated granules (16) contained in the capture enclosure (1, 1b) are picked up in order to collect them in a container (27) forming an airlock relatively to the enclosure (1, 1b).

6. The method according to any of claims 4 or 5, **characterized in that** the rate of feeding the capture enclosure (1, 1b) with the granules (16) contained in the storage container (12) is close to the pick-up rate achieved in said enclosure (1, 1b).

7. A device for capturing fine particles generated by means of a method applying a fluid at a supercritical pressure loaded with the particles, **characterized in that** it includes a capture enclosure (1, 1b) provided at its upper portion, with means for feeding particles to be captured and, at its lower portion, a bed of granules intended to fix the particles by percolation of the fluid through the bed of granules, and means for feeding and discharging a fluid at a supercritical pressure, intended for conveying said particles.

8. The capture device according to claim 7, **characterized in that** the lower portion of the capture enclosure (1, 1b) forms a downward converging hopper which is in communication with receiving/storing means (8).

9. The capture device according to claim 8, **characterized in that** the receiving/storing means (8) form an airlock relatively to the capture enclosure (1).

10. The capture device according to any of claims 8 or 9, **characterized in that** the means (6) for feeding particles to be captured form an airlock relatively to the capture enclosure (1).

## Patentansprüche

1. Verfahren zum Auffangen sehr feinteiliger Partikel, einen Schritt der Erzeugung von Partikeln mit einem Fluid mit superkritischem Druck umfassend, **dadurch gekennzeichnet, dass** das Verfahren weiterhin in einem Auffangbehälter (1, 1b) einen Perkolationsschritt durch ein aus Granulat bestehendes Aufnahmebett des mit Partikeln beladenen Fluids, die beim vorhergehenden Schritt gebildet wurden, umfasst.

2. Verfahren nach Anspruch 1, **dadurch gekennzeichnet, dass** die Partikel von einem aktiven Prinzip gebildet werden und das Granulat von einem Exzipient gebildet werden, das dazu bestimmt ist, dieses zu fixieren.

3. Verfahren nach einem der Ansprüche 1 oder 2, **dadurch gekennzeichnet, dass** ein Fluid mit superkritischem Druck verwendet wird, das von Kohlendioxid gebildet wird.

4. Verfahren nach einem der vorangehenden Absprüche, **dadurch gekennzeichnet, dass** der Auffangbehälter (1, 1b) mit Granulat (16) versorgt wird, das sich in einem Lagerbehälter (12) befinden, der im Verhältnis zum Behälter (1, 1b) eine Schleuse bildet.

5. Verfahren nach Anspruch 4, **dadurch gekennzeichnet, dass** das behandelte Granulat (16), das sich im Auffangbehälter (1, 1b) befindet, entnommen wird, um es in einem Behälter (27) gewinnen, der im Verhältnis zum Behälter (1, 1b) eine Schleuse bildet.

6. Verfahren nach einem der Ansprüche 4 oder 5, **dadurch gekennzeichnet, dass** die versorgungsleistung des Auffangbehälters (1, 1b) durch das im Lagerbehälter (12) befindliche Granulat (16) etwa der Entnahmeleistung entspricht, die in besagtem Behälter (1, 1b) erfolgt.

7. Vorrichtung zum Auffangen feiner Partikel, die mit einem Verfahren gewonnen wurden, das ein mit Partikeln beladenes Fluid mit superkritischem Druck umsetzt, **dadurch gekennzeichnet, dass** sie einen Auffangbehälter (1, 1b) umfasst, der in seinem oberen Abschnitt mit Mitteln zur Versorgung mit aufzufangenden Partikeln ausgestattet ist und in seinem unteren Abschnitt mit einem Granulatbett, das dazu bestimmt ist, die Partikel durch Perkolation des Fluids durch das Granulatbett zu fixieren, und Mittel zur Versorgung und Abführung eines Fluids mit superkritischem Druck, das dazu bestimmt ist, die Partikel zu transportieren.

8. Auffangvorrichtung nach Anspruch 7, **dadurch gekennzeichnet, dass** der untere Abschnitt des Auffangbehälters (1, 1b) einen nach unten konvergierenden Trichter bildet, der mit den Aufnahme-/Lagermitteln (8) in Kommunikation ist.

9. Auffangvorrichtung nach Anspruch 8, **dadurch gekennzeichnet, dass** die Aufnahme-/Lagermittel (8) im Verhältnis zum Auffangbehälter (1) eine Schleuse bilden.

10. Auffangvorrichtung nach einem der Ansprüche 8 oder 9, **dadurch gekennzeichnet, dass** die Mittel zur Versorgung (6) mit aufzufangenden Partikeln im Verhältnis zum Auffangbehälter (1) eine Schleuse bilden.
